# EUROPEAN PATENT SPECIFICATION

(11) **EP 2 922 569 B1**
(45) Date of publication and mention of the grant of the patent: **03.01.2018**
(21) Application number: 13856500.7
(22) Date of filing: 18.11.2013
(51) Int. Cl.: A61K 39/095, C08B 37/00, C12P 19/04

(54) **PRODUCTION OF HIGH YIELDS OF BACTERIAL POLYSACCHARIDES**
HERSTELLUNG VON BAKTERIELLEN POLYSACCHARIDEN MIT HOHER AUSBEUTE
PRODUCTION DE HAUTS RENDEMENTS DE POLYSACCHARIDES BACTÉRIENS

(30) Priority: 21.11.2012 IN 3337MU2012
(43) Date of publication of application: 30.09.2015
(73) Proprietor: Serum Institute of India Private Limited, Pune 411 028 MAH (IN)
(72) Inventor: SHANKAR, Pisal Sambhaji, Maharashtra Pune 411028 (IN); REDDY, Chilukuri Srinivas, Maharashtra Pune 411028 (IN); REDDY, Peddireddy Srinivas, Maharashtra Pune 411028 (IN)
(74) Representative: Rigamonti, Dorotea
(86) International application number: PCT/IN2013/000701
(87) International publication number: WO 2014/080423

(56) References cited:
- WO-A1-2008/102173
- WO-A1-2013/114268
- WO-A1-2013/174832
- WO-A2-03/007985
- WO-A2-2005/103230
- WO-A2-2007/084856
- CA-A1- 2 766 418
- US-A- 4 235 994
- US-A1- 2010 272 755
- US-B2- 6 933 137
- David R. Bundle ET AL: "Studies on the Group-specific Polysaccharide of Neisseria meningitidis Serogroup X and an Improved Procedure for Its Isolation", Journal of Biological Chemistry, 10 August 1974 (1974-08-10), pages 4797-4601, XP055217908, UNITED STATES Retrieved from the Internet: URL:http://www.jbc.org/content/249/15/4797 .abstract
- KENNY, CP ET AL.: 'A Chemically Defined Protein-free Liquid medium for the Cultivation of Species of Neisseria.' BULL. WORLD HEALTH ORGAN. vol. 37, no. 4, 01 January 1967, pages 569 - 573, XP002603844 Retrieved from the Internet: <URL:http://www.ncbi.nlm.nih.gov/pmc/articl es/PMC2554375/pdf/bullwho00243-0071.pdf> [retrieved on 2014-12-30]
- BUNDLE, DR ET AL.: 'Studies on the Group-specific Polysaccharide of Neisseria meningitidis Serogroup X and an Improved Procedure for Its Isolation.' J. BIOL. CHEM. vol. 249, no. 15, 10 August 1974, pages 4797 - 4801, XP055217908 Retrieved from the Internet: <URL:http://www.jbc.org/content/249/15/4797 .long> [retrieved on 2014-12-30]

## Description

### Technical Field:

The invention concerns a method for preparing a Neisseria meningitidis serogroup X (MenX) capsular polysaccharide.

### Background Art:

Neisseria meningitidis is the cause of epidemic bacterial meningitis. Capsular polysaccharide is a major virulence determinant of N. meningitidis. Among the 13 meningococcal serogroups classified based on capsular polysaccharide structure, serogroups A, B, C, Y, and W135 are associated with the majority of cases of meningococcal disease. In the African meningitis belt most large epidemics have been caused by serogroup A meningococci, whereas sporadic disease and outbreaks in developed countries are usually caused by serogroup B and C meningococci. Serogroup Y meningococci emerged as an important cause of sporadic disease and outbreaks in the United States in the late 1990s , and in 2000 serogroup W135 meningococci caused worldwide disease in association with the Hajj pilgrimage and large outbreaks in sub-Saharan Africa.

Serogroup X Neisseria meningitidis (MenX), previously a rare
cause of sporadic cases of meningitis, has recently been associated with increased incidence of meningococcal disease and has emerged as a cause of large outbreaks in the "Meningitis Belt" of Africa. Outbreaks have been documented in Niger, Burkina Faso, Togo and Ghana and have varied in size. In the meningitis season of 2010 over 6500 meningitis cases were reported in Burkina Faso, and it is reasonable to assume that at least 1000 of these cases were due to MenX with a locally reported incidence of 120 cases per 100,000. Earlier, several patients were confirmed with MenX disease in an outbreak of at least 82 cases of bacterial meningitis on the border of Kenya and Uganda in 2007.

The capsular polysaccharides of serogroup B, C, Y, and W135 meningococci are composed of sialic acid derivatives. Serogroup B and C meningococci express (a 2-8)- and (α 2- 9) - linked polysialic acid , respectively , while alternating sequences of D-glucose or D-galactose and sialic acid are expressed by serogroup Y and W135 N. meningitidis. In contrast, the capsule of serogroup A meningococci is composed of (α 1- 6)-linked N-acetylmannosamine 6-phosphate , while N. meningitidis serogroup X synthesizes capsular polymers of (α 1 -4)-linked N-acetylglucosamine 1-phosphate.

The increase in incidence of MenX disease in African Meningitis Belt in the last 5 years warrants development and introduction of a MenX vaccine in selected areas of the region to prevent and control future epidemics. The conjugation of meningococcal capsular polysaccharides to a carrier protein has led to the development of monovalent (A or C) polysaccharide conjugate vaccines with high effectiveness , and immunogenicity data from clinical trials indicate that wide use of tetravalent conjugate vaccines covering serogroups A, C, Y and W-135 may be similarly effective. A similar approach may also be fruitful for MenX.Refer Ouli Xiea et al "Characterization of size, structure and purity of serogroup X Neisseria meningitidis polysaccharide, and development of an assay for quantification of human antibodies", Vaccine 30,2012.

Gunnstein Norheim discusses that at present vaccine against serogroup X is not available and that next generation affordable vaccines should target most prevalent serogroups: A, W-135, X. Refer "Preventing the emerging serogroup X meningococcal disease in the African Meningitis Belt" Oxford Vaccine Group, 2011.

The lack of a vaccine against group X meningococci is a cause for concern given the outbreaks caused by meningococci of this serogroup in the past few years. Refer "Meningococcal vaccines: WHO position paper", November 2011.

In order to facilitate Men X polysaccharide based conjugate vaccine development, it is necessary to obtain structurally intact Men X polysaccharides with higher yields.

Several synthetic media were discovered for large-scale production of meningococcal polysaccharide (Frantz, I. D. Jr. Growth Requirements of the Meningococcus. J. Bact., 43: 757-761, 1942; Catlin, B. W. Nutritional profiles of Neisseria lactamica, gonorrhoeae and meningitidis, in chemically defined media. J. Inf. Dis., 128(2): 178-194, 1973; Watson-Scherp Medium: Watson R G, et al. The specific hapten of group C (group IIa) meningococcus, II. Chemical nature. J Immunol 1958; 81:337-44; Marcelo Fossa da Paz; Júulia Baruque-Ramos; Haroldo Hiss; Márcio Alberto Vicentin; Maria Betania Batista Leal; Isaías Raw. Polysaccharide production in batch process of Neisseria meningitidis serogroup C comparing Frantz, modified Frantz and Catlin 6 cultivation media, Braz. J. Microbiol. vol. 34., no. 1. São Paulo January/April 2003).

U.S. Pat. No. 5,494,808 reports a large-scale, high-cell density (5 g/L dry cell weight, and an optical density of between about 10-13 at 600 nm) fermentation process for the cultivation of N. meningitides (serogroup B 11). This patent disclose the following medium (called "MC.6") for culturing Neisseria meningitidis for isolation of OMPC ("Outer Membrane Protein Complex")

US 7399615 discloses a fermentation composition wherein the composition omits NH₄Cl, and an improved method of fermenting Neisseria(serogroups A,C,Y & W135) in a fermentation composition replaces ammonium chloride( nitrogen source) with a soy peptone(HSP-A; Nutricepts, Inc; Minneapolis, Minn.).The said fed batch fermentation(2L),wherein the fermentation medium as well as feed solution contains HSP-A results in Men A polysaccharide yield of about 1300 - 1400 mg/l at an average max OD between 14-20.

US 7491517 discloses a Neisseria meningitidis fastidious culture medium (NMFM) for producing capsular polysaccharides from Neisseria meningitidis(serogroups A,C,Y & W135) comprising: DI (deionizer) water, NaCl, K₂SO₄, KCI, trisodium citrate.2H₂O, MgSO₄.7H₂O, MnSO₄.H₂O, MnCl₂.6H₂O, vitamin B12, NAD (Nicotinamide Adenine Dinucleotide) thiamine HCI, soy peptone, D-glucose, L-glutamic acid, L-arginine, L-serine, L-cysteine, glycine, morpholinepropanesulphonic acid [MOPS], CaCO₃ to maintain pH at 6.5 to 7.0 and Fe₂(SO₄)₃ for serogroup A and NH₄Cl for serogroup W-135.The said fermentation results in polysaccharide yield of about 30-40 mg/L at an average max OD of 10.

David Bundle et al discusses preparation and isolation of Men X polysaccharide from N.meningitidis strain 247 X(Laboratory Center for Disease Control) wherein said strain was grown on a chemically defined medium (NCDM) for 18 hours. The said fermentation results in Men X polysaccharide yield of about 20 mg/L. Refer "Studies on the Group-specific Polysaccharide of Neisseria meningitidis Serogroup X and an improved Procedure for its isolation" JBC, 1974.

Ouli Xiea et al discloses preparation of MenX PS from strains BF7/07 and BF12/03. Briefly, the MenX strains were grown on Brain Heart Infusion agarplates with Levinthals' supplement, and following a preculture step in 0.2 L Franz medium, the strain was cultivated in four separate 2.8 L baffled shaking flasks containing 1.0 or 1.5 L modified Franz liquid medium each. Liquid cultures were inactivated after 16 h of growth by adding formaldehyde to a final concentration of 1% (v/v).MenX PS yield per liter of growth medium appeared to be slightly higher for isolate BF 7/07 (4.5 mg/L) than for isolate BF12/03 (3.8 mg/L).Refer "Characterization of size, structure and purity of serogroup X Neisseria meningitidis polysaccharide, and development of an assay for quantification of human antibodies", Vaccine 30 ,2012.

WO2005/103230 discloses a method for preparing protein-saccharide conjugate, wherein capsular saccharide is prepared from Neisseria meningitidis bacterium, inoculated in a medium comprising soy peptone as a nitrogen source.

WO2003/007985 discloses a process for purifying a bacterial capsular polysaccharide, comprising the steps of precipitating the polysaccharide and then precipitating the same using an alcohol.

Prior art discusses structural and yield improvement studies with respect to polysaccharides from serogroups Men A ,C ,Y & W135.However none of the prior art addresses the long felt need for preparation of Men X polysaccharide with higher yield which is a prerequisite for development of Men X plain polysaccharide and Men X polysaccharide protein conjugate vaccines.

Present inventors have found that prior art feeding strategies based on pH stat ,spiking and constant rate which are suitable for Men A C Y W135 fermentation ,however suffer from following setbacks for Men X :i)satisfactory growth but less polysaccharide yield ii)growth limiting , less polysaccharide yield and iii) satisfactory growth but early decline phase. Further prior art methods employ soya peptone & yeast extract as feed medium components that result in lower polysaccharide yield associated with increased load of protein contaminants.

It is an object of the present invention to provide improved culture ,fermentation and purification conditions for preparing Neisseria meningitidis X polysaccharides with high yield and minimum impurities .With said improvements, Men X polysaccharide protein conjugate vaccine manufacturing can be economical and subsequently vaccine can be made available to children of developing countries at an affordable rate.

### Summary of the Invention:

The present invention pertains to a method according to claims 1-9 for preparing a Neisseria meningitidis serogroup X (MenX) capsular polysaccharide.

Present invention arises from the surprising finding that it is possible to prepare Men X polysaccharide with high yields and high purity by utilizing a) a fermentation medium containing casamino acid along with other components, b) novel continuous exponential feeding strategy and feed medium composition that can result in delaying the decline phase, c) optimal fermenter conditions and d) improved purification process devoid of chromatographic methods.

### Brief Description of Drawings:

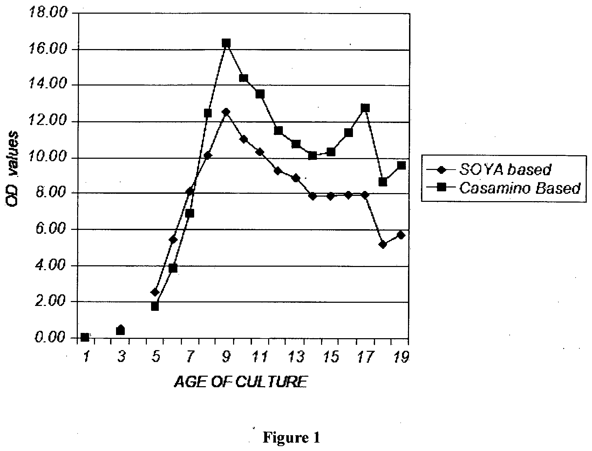

Figure 1: Growth profile of "N.meningitidis X 8210" in medium containing casamino acid in comparison with medium containing soya peptone.

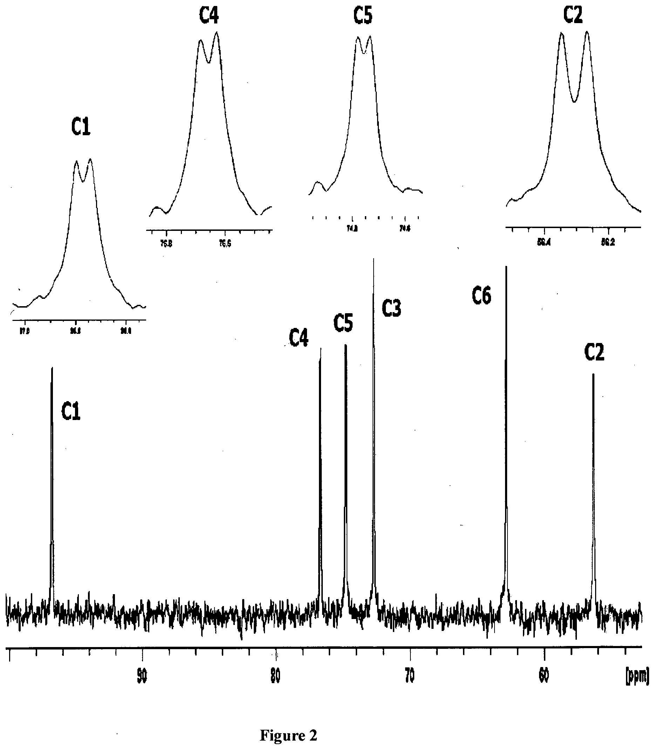

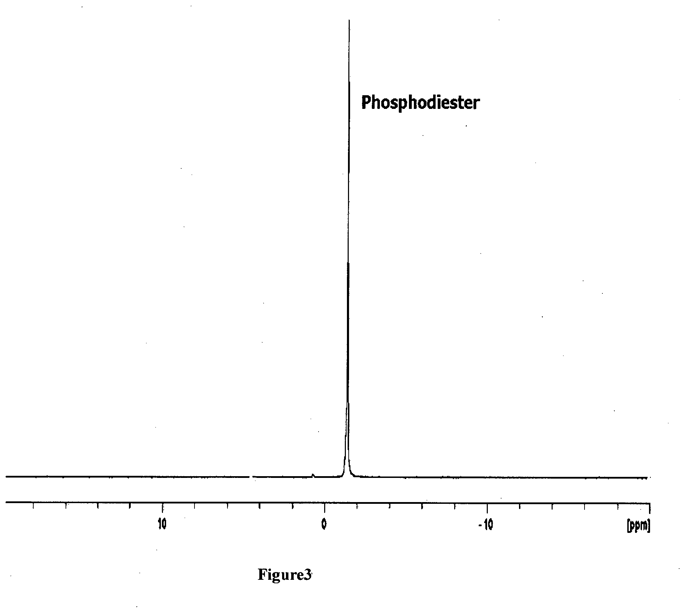

Figure 2:¹³C NMR Spectrum of Men X

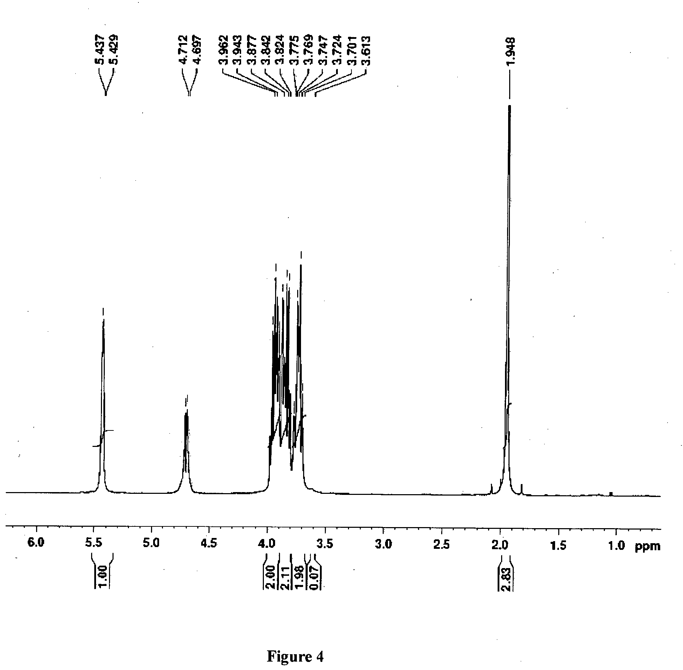

Figure 4: ³¹P NMR Spectrum of Men X
Figure 5: ¹H NMR Spectrum of Men X

### Detailed Description of Invention:

Accordingly in a first embodiment, present invention preferably utilizes fermentation medium containing casamino acid as nitrogen source instead of soya peptone or yeast extract thereby providing following advantages i)significant increase in polysaccharide yield due to delay in decline phase as a result of utilization of a novel continuous exponential feeding strategy ii) 50% decrease in concentration of protein and nucleic acid contaminants at fermenter harvest 100 KDa stage itself ,thereby ensuring that subsequent purification process can easily remove remaining impurities iii) scale up of fermentation medium containing casamino acid can be simple and economical.

A second embodiment of the instant invention is that said Neisseria meningitidis fermentation medium for producing capsular polysaccharides from Neisseria meningitidis X can comprise of dextrose between 9 and 10 g/l, sodium chloride between 5.5 and 6 g/l, potassium sulphate between 0.8 and 1 g/l, potassium phosphate dibasic between 3.5 and 4 g/l, ammonium chloride between 0.14 and 0.19 g/l, glutamic acid between 4.5 and 5.5 g/l, L -arginine between 0.2 and 0.4 g/l, L -serine between 0.4 and 0.6 g/l, L -cysteine between 0.24 and 0.26 g/l, magnesium chloride between 0.18 and 0.20, calcium chloride 0.02 g/l, ferrous sulphate 0.002g /l & casamino acid between 5 and 20 g/l.

A preferred embodiment of the instant invention is that said Neisseria meningitidis fermentation medium for producing capsular polysaccharides from Neisseria meningitidis X can comprise of dextrose 10 g/l, sodium chloride between 5.8 and 6 g/l, potassium sulphate between 0.9 and 1 g/l, potassium phosphate dibasic between 3.8 and 4 g/l, ammonium chloride between 0.14 and 0.17 g/l, glutamic acid between 4.8 and 5 g/l, L -arginine between 0.2 and 0.3 g/l, L -serine between 0.4 and 0.5 g/l, L -cysteine between 0.24 and 0.25 g/l, magnesium chloride between 0.18 and 0.19, calcium chloride 0.02 g/l, ferrous sulphate 0.002 g/l & casamino acid between 5 and 10 g/l.

An important aspect of the instant invention is that present inventors have surprisingly found an advantageous continuous exponential feeding strategy that can maintain cells in stationary phase for a longer duration ,thereby increasing N. meningitidis X polysaccharide yield ,such that the initial feed addition can be started when OD at 590nm is between 3to 4 at a rate of 10ml/hr/1.5 L which gradually increases to 30ml/hr/1.5L till the culture OD is highest and then maintained at 60ml/hr/1.5 L till culture OD reaches 50% of highest OD and then culture can be harvested.

Further inventors of present application have found that during fermentation to avoid pH drop resulting from accumulation of released cellular metabolites, NaoH at a concentration between 0.25N and 0.5N and orthophosphoric acid at a concentration between 5 and 10% can be continuously provided by the dosing pumps in cascade with the pH kept at set point, wherein ratio of sodium carbonate (20%) to NaOH (0.5N) is maintained at 1:3.

One important aspect of the instant invention is that said N. meningitidis X strain 8210 was found to have a log phase between 5^{th} and 9^{th} hr, stationary phase between 9^{th} and 12^{th} hr and decline phase between 12^{th} and 19^{th} hr.

The fermentation in the present invention is carried out in fed batch manner, preferably in a continuous fed batch mode.

In yet another aspect of the instant invention, said feed solution can comprise of dextrose between 72 and 76 g/l, sodium glutamate between 38 and 42 g/l, L -arginine between 2.8 and 3.2 g/l, L-serine between 2.8 and 3.2 g/l, L- cysteine between 1.9 and 2.1 g/l, magnesium chloride between 1.9 and 2.1, calcium chloride between 0.13 and 0.15 g/l and ferrous sulphate 0.02g /l .

In a third embodiment, present invention provides a Men X capsular polysaccharide having yield between 600 mg/l to 800 mg/l at 100 KDa fermenter harvest stage wherein optical density measured at 590 nm can be between 8 and 15 , preferably between 8 and 11.

A third embodiment of the present invention is that fermentation of Neisseria meningitidis X can be performed at set point values of i) pH from 7 to about 7.2,ii)temperature between 36 and 37°C and at cascading values for i) dissolved oxygen from 15 to 25%,ii)agitation from 350-500 rpm, iii) Gas flow from 1 to 1.5,iv)Air from 0 to 100% and v)Oxygen from 0 to 100%.

A fourth embodiment of the instant invention is that the 100KDa diafiltration harvest can be further subjected to purification steps comprising of:
(a) removal of protein and endotoxin impurities by utilizing deoxycholate at a concentration of 1% in combination with ethylenediaminetetraacetic acid at a concentration of 2mM & ethanol at a concentration of 40%;
(b) addition of 4 to 6% sodium acetate for removal of nucleic acids;
(c) addition of cetyltrimethylammonium bromide at a concentration from 3 to 4% for binding polysaccharide and impurities;
(d) precipitation of polysaccharide from cetyltrimethylammonium bromide-polysaccharide complex by utilizing sodium chloride at a concentration of 0.05 M in presence of 96% absolute ethanol;
(e) removal of protein and nucleic acid impurities by washing pellet with ethanol at a concentration of 45% in presence of sodium chloride at a concentration of 0.4 M;
(f) selective precipitation of polysaccharide by utilizing 96 % absolute ethanol;
(g) dissolving polysaccharide in WFI and subjecting to tangential flow filtration; and
wherein said purification process does not utilize any chromatography and said purified polysaccharide has yield from 300 to 500 mg/L ,average molecular weight from 400 to 550 KDa, contains less than 0.5% proteins/peptides, less than 0.5% nucleic acids ,less than 5 EU/µg endotoxins with purification step recovery from 60% to 65%.

Another aspect of the fourth embodiment is that said N. meningitidis X polysaccharide purification process is robust and cost-efficient as it provides about 60 to 70 % polysaccharide recovery and does not require any additional chromatographic steps.

It is here described that said process can be applicable to N. meningitidis serotype X strains selected from M9601, M9592,M9591,247X, M9554, M8210 and M2526 ,5967 strain (ST 750),most preferably to"M8210".

A sixth embodiment of the instant invention is that said N. meningitidis X polysaccharide of the instant invention can be utilized to prepare polysaccharide protein conjugate composition by methods disclosed in WO 201314268 wherein, i) polysaccharide X can be sized mechanically to obtain fragments having size between 150 and 200 KDa, ii) sized saccharide can be conjugated to carrier protein via a linker with a cyanylation conjugation chemistry iii) saccharide to protein ratio in final conjugate can be between 0.2 - 0.6.

It is here described that said N. meningitidis X polysaccharide of the instant invention can also be utilized to prepare a multivalent meningococcal polysaccharide protein conjugate composition comprising capsular saccharide from serogroups X and at least one additional capsular polysaccharide from A, C, W135 and Y by methods disclosed previously in WO 201314268.

Seventh embodiment of present invention is that said carrier protein can be selected from a group of but not limited to CRM 197,diphtheria toxoid, tetanus toxoid, pertussis toxoid, E. coli LT, E: coli ST, and exotoxin A from Pseudomonas aeruginosa, outer membrane complex c (OMPC), porins, transferrin binding proteins, pneumolysin, pneumococcal surface protein A (PspA), pneumococcal adhesin protein (PsaA), pneumococcal surface proteins BVH-3 and BVH-11 , protective antigen (PA) of Bacillus anthracis and detoxified edema factor (EF) and lethal factor (LF) of Bacillus anthracis, ovalbumin, keyhole limpet hemocyanin (KLH), human serum albumin, bovine serum albumin (BSA) and purified protein derivative of tuberculin (PPD).Preferably, carrier proteins can be selected from tetanus toxoid, diphtheria toxoid and CRM197.

Monovalent or multivalent immunogenic compositions containing N. meningitidis X polysaccharide can be in a buffered liquid form or in a lyophilized form. Preferably,said polysaccharide protein conjugate can be lyophilized as disclosed previously in US2013/0209503, wherein the formulation can have at least 6 month stability at 40°C and free polysaccharide content can be less than 11% w/w.

The lyophilized vaccine composition can be reconstituted with a delivery vehicle having pH from about 6 to 7.5,particularly with saline or PBS.

Compositions can comprise of aluminium salt adjuvant added at an amount of 25-125µg of Al⁺⁺⁺ per 0.5 ml. Also said composition can comprise of a preservative selected from thiomersal and 2-phenoxyethanol.

The lyophilized vaccine composition can be given as 1,5 or 10 dose formulation.

The polysaccharide or a conjugate thereof is preferably administered parenterally, e.g. by injection or infusion by intravenous, intraperitoneal, intramuscular, intraarterial or intralesional route.

### Examples:

### I) Fermentation Procedure:

Seed vial containing 3 ml of "N. meningitidis X M8210"(CBER)culture having OD 1/ml was freezed at -70°C. Then vial was thawed and seeded into 30 ml of seed medium which was incubated at 37 °C and agitated at 150 to 180 rpm. The volume was doubled to 60 ml when OD was above 0.7 and then to 120 ml , 210 ml respectively.

Culture having OD 1.0 ± 0.2 with volume 70 ± 10 ml was seeded into the reactor, wherein the medium volume in the reactor was 1500 ml. After inoculation of reactor 0 hr OD was maintained at 0.04 to 0.05.

The entire fermentation process was carried out in NBS bio flow Celligen 115 (2.2L) glass bioreactor, wherein fermentation cycle was run in a continuous fed batch mode and total duration of fermentation cycle was 19hrs .

### II) Fermentation medium composition:

**Table 1:Composition of novel medium containing casamino acid for N. meningitidis serogroup X (NMXM-CA-I)**

| **Component** | **Concentration (gm/l)** |
|---|---|
| Dextrose | 10 |
| Sodium chloride | 5.8 |
| Potassium sulphate | 1 |
| Potassium phosphate dibasic | 4 |
| Ammonium chloride | 0.15 |
| Glutamic acid | 5 |
| Arginine | 0.3 |
| Serine | 0.5 |
| Cysteine | 0.25 |
| Magnesium chloride | 0.19 |
| Calcium chloride | 0.02 |
| Ferrous sulphate | 0.002 |
| Casamino acid | 10 |

**Table 2:Composition of novel medium containing casamino acid for N. meningitidis serogroup X (NMXM-CA-II)**

| **Component** | **Concentration (gm/l)** |
|---|---|
| Dextrose | 10 |
| Sodium chloride | 5.8 |
| Potassium sulphate | 1 |
| Potassium phosphate dibasic | 4 |
| Ammonium chloride | 0.15 |
| Glutamic acid | 5 |
| Arginine | 0.3 |
| Serine | 0.5 |
| Cysteine | 0.25 |
| Magnesium chloride | 0.19 |
| Calcium chloride | 0.02 |
| Ferrous sulphate | 0.002 |
| Casamino acid | 8 |

**Table 3:Composition of novel medium containing casamino acid for N. meningitidis Serogroup X(NMXM-CA-III)**

| **Component** | **Concentration (gm/l)** |
|---|---|
| Dextrose | 10 |
| Sodium chloride | 5.8 |
| Potassium sulphate | 1 |
| Potassium phosphate dibasic | 4 |
| Ammonium chloride | 0.15 |
| Glutamic acid | 5 |
| Arginine | 0.3 |
| Serine | 0.5 |
| Cysteine | 0.25 |
| Magnesium chloride | 0.19 |
| Calcium chloride | 0.02 |
| Ferrous sulphate | 0.002 |
| Casamino acid | 7 |

**Table 4:Composition of a medium containing soya peptone for N. meningitidis serogroup X (NMXM-SP)**

| **Component** | **Concentration (gm/l)** |
|---|---|
| Dextrose | 10 |
| Sodium chloride | 5.8 |
| Potassium sulphate | 1 |
| Potassium phosphate dibasic | 4 |
| Ammonium chloride | 0.15 |
| Glutamic acid | 5 |
| Arginine | 0.3 |
| Serine | 0.5 |
| Cysteine | 0.25 |
| Magnesium chloride | 0.19 |
| Calcium chloride | 0.02 |
| Ferrous sulphate | 0.002 |
| Soya peptone | 9 |

**Table 5:Composition of a medium containing soya peptone for N. meningitidis serogroup X (NMXM-SP-II)**

| **Component** | **Concentration (gm/l)** |
|---|---|
| Dextrose | 10 |
| Sodium chloride | 5.8 |
| Potassium sulphate | 1 |
| Potassium phosphate dibasic | 4 |
| Ammonium chloride | 0.15 |
| Glutamic acid | 5 |
| Arginine | 0.3 |
| Serine | 0.5 |
| Cysteine | 0.25 |
| Magnesium chloride | 0.19 |
| Calcium chloride | 0.02 |
| Ferrous sulphate | 0.002 |
| Soya peptone | 8 |

### III) Growth Kinetics, Novel Feeding Strategy & Feed solutions:

**Table 6:Growth profile of N. meningitidis X strain 8210**

| **Age** | **OD (590nm)in medium containing *casamino acid*** | **OD (590nm)in medium containing *soya peptone*** |
|---|---|---|
| 0 | 0.07 | 0.08 |
| 1 | 0.19 | 0.23 |
| 2 | 0.40 | 0.55 |
| 3 | 0.80 | 1.27 |
| 4 | 1.73 | 2.55 |
| 5 | 3.89 | 5.49 |
| 6 | 6.93 | 8.17 |
| 7 | 12.45 | 10.08 |
| 8 | 16.40 | 12.49 |
| 9 | 14.40 | 11.03 |
| 10 | 13.49 | 10.33 |
| 11 | 11.48 | 9.28 |
| 12 | 10.74 | 8.84 |
| 13 | 10.09 | 7.89 |
| 14 | 10.29 | 7.92 |
| 15 | 10.32 | 7.97 |
| 16 | 11.32 | 7.94 |
| 17 | 11.67 | 5.21 |
| 18 | 11.54 | 5.77 |

The log phase was identified between 5^{th} and 9^{th} hr, stationary phase between 9^{th} and 12^{th} hr and decline phase between 12^{th} and 19^{th} hr.

### Novel Feeding strategy:

The feeding strategy was designed such that the initial feed addition can be started when OD at 590nm is between 3to 4 at a rate of 10ml/hr/1.5 L which gradually increases to 30ml/hr/1.5L till the culture OD is highest and then maintained at 60ml/hr/1.5 L till culture OD reaches 50% of highest OD and then culture can be harvested.

### Feed solutions:

**Table 7:Composition of novel "Feed Solution 1"(FS-1) for N. meningitidis serogroup X**

| **Component** | **Concentration (gm/l)** |
|---|---|
| Dextrose | 75 |
| Sod glutamate | 40 |
| Arginine | 3 |
| Serine | 3 |
| Cysteine | 2 |
| Magnesium chloride | 2 |
| Calcium chloride | 0.15 |
| Ferrous sulphate | 0.02 |

**Table 8:Composition of novel "Feed Solution 2"(FS-2) for N. meningitidis serogroup X**

| **Component** | **Concentration (gm/l)** |
|---|---|
| Dextrose | 75 |
| Sod glutamate | 40 |

### Results:

Continuous exponential feeding strategy resulted in maintaining cells in stationary phase for a longer duration, thereby increasing yield of N. meningitidis X polysaccharide .Use of Casamino acid as a nitrogen source was found to provide high yield & high molecular weight Men X polysaccharide with minimal impurities at 100 KDa stage as compared to soya peptone as nitrogen source.

When only dextrose and sodium glutamate were used as feed solution (FS-2), cell morphology was affected ultimately resulting in unfavorable polysaccharide characteristics. Whereas when FS-1 (containing amino acids , salts in addition to dextrose and sodium glutamate) was used morphology of cells was found to be improved , thereby increasing polysaccharide yield and providing polysaccharide with favorable characteristics.

Study of growth profile of N. meningitidis X 8210 reveals that during fermentation a pH drop resulting from accumulation of released cellular metabolites was observed that was adversely affecting rate of capsular polysaccharide production. To avoid such pH drop , NaOH at a concentration between 0.25N and 0.5N and orthophosphoric acid at a concentration between 5 and 10% was continuously provided by the dosing pumps in cascade with the pH kept at set point, wherein ratio of sodium carbonate (20%) to NaOH (0.5N) is maintained at 1:3.

### IV) Fermenter conditions

**Table 9: Fermenter variables**

| **Fermentation aspect** | **Concentration** |
|---|---|
| Temperature | 36 °C |
| OD | 16 OD |
| pH | 7.2 |
| DO(%) | 25% |
| Agitation(rpm) | 400 |
| Gas flow | 1.0 |
| Air(%) | 0-100% |
| Oxygen(%) | 0-100% |

**Table 10: Polysaccharide Concentration & purity profile at 100 KDa**

| **Nitrogen sources utilized** | **Polysaccharide concentration (mg/l)** | **Relative concentrations of Endotoxin , Protein & Nucleic acid** |
|---|---|---|
| Men X Ps at 100 KDa fermentation harvest stage for NMXM-CA-I | 650 | Protein :50- 75 % |
| | | Nucleic acid: 15-30% |
| Men X Ps at 100 KDa fermentation harvest stage for NMXM-CA-II | 625 | Protein :42- 70 % |
| | | Nucleic acid: 15-30% |
| Men X Ps at 100 KDa fermentation harvest stage for NMXM-CA-III | 610 | Protein :35- 65 % |
| | | Nucleic acid: 15-30% |
| Men X Ps at 100 KDa fermentation harvest stage for NMXM-SP-I | 500 | Protein :65- 95 % |
| | | Nucleic acid: 15-40% |
| Men X Ps at 100 KDa fermentation harvest stage for NMXM-SP-II | 475 | Protein :65- 90 % |
| | | Nucleic acid: 15-40% |

### Results:

For NMXM-CA medium containing casamino acids, at 100 KDa stage, polysaccharide yield was between 500 and 650 mg/l with minimal load of wherein harvesting was carried at when OD reached 50% of highest culture OD. Whereas for NMXM-SP medium containing soya peptone, at 100 KDa stage, polysaccharide yield was comparatively low i.e. between 450 and 500 mg/l with greater load of impurities.

### V) Harvesting & Inactivation

The fermentation was terminated once drop in optical density was observed followed by inactivation using 1% formaldehyde for 2hrs at 37°C. Further the temperature was reduced at 10°C and incubated for 30 minutes. The harvest was unloaded and centrifuged at 14,500g for 45 minutes.

Then supernatant was subjected to 0.2µ filtration followed by 100KD diafiltration (10-15 times) and was further concentrated with WFI. Later sterile filtration with 0.2 µ filter was carried.

### VI) Purification of Men X polysaccharide

### Protocol 1:

Addition of 0.5% deoxycholate , 6% sodium acetate, 2mM EDTA & 40% ethanol to the 100KD diafiltered harvest. Then the mixture was kept at 2-8°C for 3-4hrs with stirring. Later mixture was subjected to centrifugation at 10000 rpm for 20min and supernatant was diafiltered against 25 mM Tris with 100KD cassette membrane. Further 10% w/v CTAB precipitation was carried overnight at 2-8°C with stirring and pellet was collected. Said pellet was dissolved in 96% ethanol with 0.05M NaCl at 2-8°C for 2 hrs on stirring. Then polysaccharide precipitation was carried for 30 minutes and pellet was collected. Said pellet was dissolved in 45% ethanol with 0.4M NaCl for 1hr. The supernatant was collected & filtered through CUNO R32SP carbon filter. Then polysaccharide was precipitated in 96 % ethanol for 1-2 hrs and pellet was collected. Then pellet was dissolved in WFI followed by TFF. Final purified N. meningitidis X polysaccharide was stored at -20° C.

### Protocol 2:

Addition of 1.5% deoxycholate , 6% sodium acetate, 2mM EDTA & 40% ethanol to the 100KD diafiltered harvest. Then the mixture was kept at 2-8°C for 3-4hrs with stirring. Later mixture was subjected to centrifugation at 10000 rpm for 20min and supernatant was diafiltered against 25 mM Tris with 100KD cassette membrane. Further 6% w/v CTAB precipitation was carried overnight at 2-8°C with stirring and pellet was collected. Said pellet was dissolved in 96% ethanol with 0.05M NaCl at 2-8°C for 2 hrs on stirring. Then polysaccharide precipitation was carried for 30 minutes and pellet was collected. Said pellet was dissolved in 45% ethanol with 0.4M NaCl for 1hr. The supernatant was collected & filtered through CUNO R32SP carbon filter. Then polysaccharide was precipitated in 96 % ethanol for 1-2 hrs and pellet was collected. Then pellet was dissolved in WFI followed by TFF. Final purified N. meningitidis X polysaccharide was stored at -20° C.

### Protocol 3:

Addition of 1% deoxycholate , 6% sodium acetate, 2mM EDTA & 40% ethanol to the 100KD diafiltered harvest. Then the mixture was kept at 2-8°C for 3-4hrs with stirring. Later mixture was subjected to centrifugation at 10000 rpm for 20min and supernatant was diafiltered against 25 mM Tris with 100KD cassette membrane. Further 3% w/v CTAB precipitation was carried overnight at 2-8°C with stirring and pellet was collected. Said pellet was dissolved in 96% ethanol with 0.05M NaCl at 2-8°C for 2 hrs on stirring. Then polysaccharide precipitation was carried for 30 minutes and pellet was collected. Said pellet was dissolved in 45% ethanol with 0.4M NaCl for 1hr. The supernatant was collected & filtered through CUNO R32SP carbon filter. Then polysaccharide was precipitated in 96 % ethanol for 1-2 hrs and pellet was collected. Then pellet was dissolved in WFI followed by TFF. Final purified N. meningitidis X polysaccharide was stored at -20° C.

Protocol 1 having low DOC resulted caused inefficient removal of contaminants (protein/nucleic acids) whereas & high CTAB concentrations resulted in a complex of CTAB-polysaccharide that was not readily separable.

Protocol 2 having higher DOC resulted in more viscous polysaccharide solutions thus making it difficult for TFF processing. Also intermediate CTAB concentration resulted in a complex of CTAB-polysaccharide that was not readily separable.

Protocol 3 having intermediate DOC and less CTAB concentration was found to be more efficient than Protocol 1/2 and hence was finalized for purification of N. meningitidis X polysaccharide.

The purified N. meningitidis X polysaccharide prepared by Protocol 3 was tested for impurities like Protein, Nucleic acid , Endotoxin and Molecular size .Although WHO specification / guidelines for Men X polysaccharide were not available, purified N. meningitidis X polysaccharide was found to meet WHO specifications already set for a similar phosphodiester containing polysaccharide i.e. N. meningitidis A.

Further, comparison of ¹H, ¹³C, ³¹P NMR assignments of SIIL's Men X polysaccharide with recently published X-related NMR data (Vaccine 30 ,2012, 5812-5823 & Vaccine 30, 2012, 6409-6415) confirm structural identity of SIIL's Men X polysaccharide.

**Table 11: Characteristics of Purified Men X polysaccharide**

| **Nitrogen sources utilized** | **Ps Conc (mg/l)** | **Nucleic acid (%)** | **Endotoxin (Eu/ug)** | **Protein(%)** | **Mw(KDa)** |
|---|---|---|---|---|---|
| Men X Ps for NMXM-CA-I | 440 | 0.062 | 1.51 | 0.18 | 410 |
| Men X Ps for NMXM-CA-II | 420 | 0.047 | 1.33 | 0.14 | 520 |
| Men X Ps for NMXM-CA-III | 380 | 0.052 | 1.25 | 0.13 | 549 |
| Men X Ps for NMXM-SP-I | 300 | 0.081 | 1.90 | 0.19 | 380 |
| Men X Ps for NMXM-SP-II | 250 | 0.062 | 1.25 | 0.15 | 463 |

### Results:

Both, NMXM-CA NMXM-SP based batches of Men X polysaccharide were found to meet WHO specifications set for N. meningitidis A polysaccharide.

For NMXM-CA medium containing casamino acids, at final purified stage, purification step recovery was between 60 and 65%,polysaccharide yield was between 350 and 500 mg/l with minimal load of impurities when harvesting was carried when culture OD reaches 50% of highest culture OD. Whereas for NMXM-SP medium containing soya peptone, at final purified stage, purification step recovery for polysaccharide as well as polysaccharide yield was comparatively low along with greater load of impurities.

Above high Men X polysaccharide yield was achieved without utilizing any additional chromatographic methods, thereby demonstrating that above purification protocol is robust & cost-efficient.

### VII) Preparation of Lyophilized, immunogenic MenX polysaccharide-protein conjugates:

Monovalent or multivalent immunogenic compositions containing N. meningitidis X polysaccharide-tetanus toxoid can be in a buffered liquid form or in a lyophilized form.

Preferably,said polysaccharide protein conjugate can be lyophilized as disclosed previously in US 2013/0209503,wherein the formulation can have at least 6 month stability at 40°C and free polysaccharide content can be less than 11 % w/w.

Men X polysaccharide of the instant invention can be utilized to prepare immunogenic ,multivalent meningococcal polysaccharide protein conjugate compositions comprising capsular saccharide from serogroups X and at least one capsular saccharide from A, C, W135 and Y as discussed previously in WO 201314268.

## Claims

1. A method for preparing a Neisseria meningitidis serogroup capsular polysaccharide (Neisseria meningitis X) comprising the steps of:
(a) preparing an aqueous fermenter nutrient medium comprising casamino acids
(b) inoculating the fermenter nutrient medium with a Neisseria meningitidis X bacterium having optical density (OD) at 590nm of 0.8 to 1.2
(c) continuous exponential feeding with a feed solution at a feed addition rate varying between 10ml/hr/1.5L and 60ml /hr/1.5 L beginning at OD between 3 and 4;
(d) incubating the inoculated fermenter nutrient medium under controlled conditions of pH, temperature and dissolved oxygen percentage;
(e) harvesting the capsular saccharide produced in step (d) when optical density(OD)at 590nm is less than 60% of highest culture OD;
(f) purifying the capsular saccharide obtained in step (e);
(g)optionally concentrating the purified capsular polysaccharide obtained in step (f);
and wherein said purified polysaccharide has yield from 300 to 550 mg/L ,average molecular weight from 400 to 550 KDa, purification step recovery from 60% to 65% and contains less than 0.5% proteins/peptides, less than 0.5% nucleic acids and less than 5 EU/µg endotoxins.

2. A method for the production of Neisseria meningitidis X capsular polysaccharide as claimed in claim 1 wherein:
(a) said capsular polysaccharide producing bacteria is selected from N.meningitidis X strains M8210, M9601,M9591, M9592, M9554,M2526,247X and 5967(ST 750);
(b) said fermenter nutrient medium comprises of:
(i)casamino acids at an amount 5g/L to 20 g/L ,0.14g/L to 0.19 g/L Ammonium chloride , 10 g/L to 11 g/L dextrose, ,5.8g/L to 6 g/L Sodium chloride ,0.9g/L to 1 g/L Potassium sulphate ,3.8g/L to 4 g/L Potassium phosphate dibasic , 4.8g/L to 5 g/L Glutamic acid ,0.2g/L to 0.3 g/l L- Arginine, 0.4g/L to 0.5 g/L L-Serine,0.24g/L to 0.25 g/L L -Cysteine ,0.18 g/L to 0.19 g/L Magnesium chloride ,0.02 g/l Calcium chloride and 0.002g /L Ferrous sulphate such that concentration of components of the medium may vary by ± 10% or
(ii)casamino acids at an amount 5g/L to 10 g/L ,0.14g/L to 0.17 g/L Ammonium chloride , 10 g/L to 11 g/L dextrose, ,5.8g/L to 6 g/L Sodium chloride ,0.9g/L to 1 g/L Potassium sulphate ,3.8g/L to 4 g/L Potassium phosphate dibasic , 4.8g/L to 5 g/L Glutamic acid ,0.2g/L to 0.3 g/l L- Arginine, 0.4g/L to 0.5 g/L L-Serine,0.24g/L to 0.25 g/L L -Cysteine ,0.18 g/L to 0.19 g/L Magnesium chloride ,0.02 g/l Calcium chloride and 0.002g /L Ferrous sulphate such that concentration of components of the medium may vary by ± 10%
(c) said inoculation of fermenter nutrient medium is by a seed innoculum having an optical density of 0.8 to 1 ;
(d) said continuous exponential feeding begins with initial feed addition at a rate of 10ml/hr/1.5 L when OD at 590nm is between 3 to 4 which gradually increases to 30ml/hr/1.5L till the culture OD is highest and then maintained at 60ml/hr/1.5 L till culture OD reaches 50% of highest OD when culture can be harvested;
(e) said feed solution comprises of 72 to 76 g/L dextrose ,38 g/L to 42 g/L Sodium glutamate , 2.8g/L to 3.2 g/L L-Arginine ,2.8 g/L to 3.2 g/L L-Serine ,1,9g/L to 2.1 g/l L- Cysteine,1.9g/L to 2g/L Magnesium chloride ,0.13g/L to 0.15 g/l Calcium chloride and 0.02g /L Ferrous sulphate such that concentration of components of the feed solution may vary by ±10%.
(f) said incubation is carried out
(i) at a temperature of 36°C to 37° C, at a pH of 7-7.2 ,at 350 to 500 rpm , dissolved oxygen percentage of 15 % to 25% ,Gas flow at 1 to 1.5, Air at 0 to 100% and Oxygen at 0 to 100% for 7 to 10 hours or
(ii) at a temperature of 36°C, at a pH 7.2 ,at about 400 rpm ,dissolved oxygen percentage of 25%,Gas flow at 1 to 1.5, Air at 0 to 100% and Oxygen at 0 to 100% for 8 to 9 hours;
(g) inactivation is carried using 1% formaldehyde for 1 to 2hrs at 37°C followed by incubation at 10°C for 30 minutes;
(h) said harvesting is carried out by
(i) centrifugation at 14500 g for 45 minutes to remove all cell residues;
(ii) subjecting supernatant to 0.2µ filtration followed by 100KD diafiltration and concentration with WFI (Water For Injection); and
(i) said method is carried out in continuous fed-batch culture system

3. A method of claim 1 wherein step (e) comprises:
(a) removal of protein and endotoxin impurities by utilizing anionic detergent at a concentration from 1 to 1.2% in combination with chelating agent at a concentration from 1.5 to 3mM and alcohol at a concentration from 40% to 50%;
(b) addition of sodium acetate at a concentration from 4 to 6% for removal of nucleic acids;
(c) addition of a cationic detergent at 3 to 4% for binding polysaccharide and impurities;
(d) precipitation of polysaccharide from cationic detergent-polysaccharide complex by utilizing sodium chloride at 0.05 M in presence of 96% alcohol;
(e) removal of protein and nucleic acid impurities by washing pellet with 45 % alcohol in presence of sodium chloride at a concentration of 0.4 M;
(f) selective precipitation of polysaccharide by utilizing 96 % alcohol;
(g) dissolving polysaccharide in WFI and subjecting to tangential flow filtration;and
wherein said purification process does not utilize any chromatography.

4. A method of claim 3, wherein purification comprises of following steps:
(a) removal of protein and endotoxin impurities by utilizing deoxycholate at a concentration of 1% in combination with ethylenediaminetetraacetic acid at a concentration of 2mM & ethanol at a concentration of 40%;
(b) addition of 4 to 6% sodium acetate for removal of nucleic acids;
(c) addition of cetyltrimethylammonium bromide at a concentration from 3 to 4% for binding polysaccharide and impurities;
(d) precipitation of polysaccharide from cetyltrimethylammonium bromide-polysaccharide complex by utilizing sodium chloride at a concentration of 0.05 M in presence of 96% ethanol;
(e) removal of protein and nucleic acid impurities by washing pellet with ethanol at a concentration of 45% in presence of sodium chloride at a concentration of 0.4 M;
(f) selective precipitation of polysaccharide by utilizing 96 % ethanol;
(g) dissolving polysaccharide in WFI and subjecting to tangential flow filtration;and
wherein said purification process does not utilize any chromatography.

5. The method according to claim 1, further comprising step (h) wherein purified N.meningitidis X polysaccharide is sized to an average size of between 100 and 150 Kda by using high pressure cell disruption system.

6. The method according to claim 5 further comprising step (i) wherein said size reduced polysaccharide is conjugated to a carrier protein, to give a protein-saccharide conjugate.

7. The method according to claim 6, wherein said N. meningitidis X saccharide is conjugated to a carrier protein selected from the group consisting of TT, DT, CRM197, fragment C of TT, protein D, OMPC and pneumolysin.

8. The method according to claim 6, wherein said N. meningitidis X saccharide is conjugated to the carrier protein via a hetero or homo-bifunctional linker with cyanylation conjugation chemistry.

9. The method according to claim 8, wherein said cyanylation reagent is selected from a group of I-cyano- 4-pyrrolidinopyridinium tetrafluoroborate (CPPT), 1- cyano- imidazole (1-CI), 1-cyanobenzotriazole (1-CBT), or 2-cyanopyridazine -3(2H) one (2-CPO).

## Patentansprüche

1. Verfahren zur Herstellung eines Neisseria-meningitidis-Serogruppen-Kapsel-Polysaccharids (Neisseria meningitis X) umfassend die Schritte:
(a) Herstellen eines wässrigen Fermenter-Nährmediums umfassend Casaminosäuren;
(b) Impfen des Fermenter-Nährmediums mit einem Neisseria-meningitidis-X-Bakterium mit einer optischen Dichte (OD) bei 590 nm von 0,8 bis 1,2;
(c) kontinuierliches exponentielles Füttern mit einer Fütterlösung bei einer Futter-Zugaberate, welche zwischen 10 ml/h/1,5 l und 60 ml/h/1,5 l, beginnend bei OD zwischen 3 und 4, variiert;
(d) Inkubieren des geimpften Fermenter-Nährmediums unter kontrollierten Bedingungen von pH, Temperatur und prozentualer Menge von gelöstem Sauerstoff;
(e) Ernten der Kapsel-Saccharide, die in Schritt (d) erzeugt wurden, wenn die optische Dichte (OD) bei 590 nm weniger als 60 % der höchsten Kultur-OD beträgt;
(f) Reinigen des in Schritt (e) erhaltenen Kapsel-Saccharids;
(g) optional Konzentrieren des in Schritt (f) erhaltenen gereinigten Kapsel-Polysaccharids;
und worin das gereinigte Polysaccharid in einer Ausbeute von 300 bis 550 mg/l, einem durchschnittlichen Molekulargewicht von 400 bis 550 KDa, einer Reinigungsschrittwiederfindung von 60 % bis 65 % und enthaltend weniger als 0,5 % Proteine/Peptide, weniger als 0,5 % Nukleinsäuren und weniger als 5 EU/µg Endotoxine vorliegt.

2. Verfahren zur Herstellung eines Neisseria-meningitidis-X-Kapsel-Polysaccharids gemäß Anspruch 1, worin:
(a) das Kapsel-Polysaccharid erzeugende Bakterium ausgewählt ist aus N.mengingitidis X Stämmen M8210, M9601, M9591, M9592, M9554, M2526, 247X und 5967 (ST 750);
(b) das Fermenter-Nährmedium umfasst:
(i) Casaminosäuren in einer Menge von 5 g/l bis 20 g/l, 0,14 g/l bis 0,19 g/l Ammoniumchlorid, 10 g/l bis 11 g/l Dextrose, 5,8 g/l bis 6 g/l Natriumchlorid, 0,9 g/l bis 1 g/l Kaliumsulfat, 3,8 g/l bis 4 g/l Dikaliumhydrogenphosphat, 4,8 g/l bis 5 g/l Glutaminsäure, 0,2 g/l bis 0,3 g/l L-Arginin, 0,4 g/l bis 0,5 g/l L-Serin, 0,24 g/l bis 0,25 g/l L-Cystein, 0,18 g/l bis 0,19 g/l Magnesiumchlorid, 0,02 g/l Kalziumchlorid und 0,002 g/l Eisensulfat in solcher Form, dass die Konzentration der Komponenten des Mediums um ± 10 % variieren kann oder
(ii) Casaminosäuren in einer Menge von 5 g/l bis 10 g/l, 0,14 g/l bis 0,17 g/l Ammoniumchlorid, 10 g/l bis 11 g/l Dextrose, 5,8 g/l bis 6 g/l Natriumchlorid, 0,9 g/l bis 1 g/l Kaliumsulfat, 3,8 g/l bis 4 g/l Dikaliumhydrogenphosphat, 4,8 g/l bis 5 g/l Glutaminsäure, 0,2 g/l bis 0,3 g/l L-Arginin, 0,4 g/l bis 0,5 g/l L-Serin, 0,24 g/l bis 0,25 g/l L-Cystein, 0,18 g/l bis 0,19 g/l Magnesiumchlorid, 0,02 g/l Kalziumchlorid und 0,002 g/l Eisensulfat, in solcher Weise, dass die Konzentration der Komponenten des Mediums um ± 10 % variieren kann,
(c) die Impfung des Fermenter-Nährmediums mittels einer Impfkultur mit einer optischen Dichte von 0,8 bis 1 durchgeführt wird;
(d) das kontinuierliche exponentielle Füttern mit einer anfänglichen Futterzugabe bei einer Rate von 10 ml/h/1,5 l beginnt, wenn die OD bei 590 nm zwischen 3 und 4 liegt, und allmählich erhöht wird auf 30 ml/h/1,5 l, bis die OD der Kultur am höchsten ist, und dann gehalten wird bei 60 ml/h/1,5 l, bis die OD der Kultur 50 % der höchsten OD beträgt, zu welchem Zeitpunkt die Kultur geerntet werden kann;
(e) die Nährlösung 72 bis 76 g/l Dextrose, 38 g/l bis 42 g/l Natriumglutamat, 2,8 g/l bis 3,2 g/l L-Arginin, 2,8 g/l bis 3,2 g/l L-Serin, 1,9 g/l bis 2,1 g/l L-Cystein, 1,9 g/l bis 2 g/l Magnesiumchlorid, 0,13 g/l bis 0,15 g/l Kalziumchlorid und 0,02 g/l Eisensulfat umfasst, in solcher Weise, dass die Konzentration der Komponenten der Nährlösung um ± 10 % variieren kann;
(f) die Inkubation ausgeführt wird
(i) bei einer Temperatur von 36°C bis 37°C, bei einem pH von 7 bis 7,2, bei 350 bis 500 U/min, einem prozentualen Anteil an gelöstem Sauerstoff von 15 bis 25 %, einem Gasfluss von 1 bis 1,5, Luft mit 0 bis 100 % und Sauerstoff mit 0 bis 100 % über 7 bis 10 Stunden oder
(ii) bei einer Temperatur von 36°C, bei einem pH von 7,2, bei ungefähr 400 U/min, bei einem prozentualen Gehalt an gelöstem Sauerstoff von 25 %, Gasfluss von 1 bis 1,5,
Luft bei 0 bis 100 % und Sauerstoff bei 0 bis 100 % für 8 bis 9 Stunden;
(g) Inaktivierung ausgeführt wird unter Verwendung von 1 % Formaldehyd für 1 bis 2 Stunden bei 37°C gefolgt von Inkubation bei 10°C für 30 Minuten;
(h) das Ernten ausgeführt wird durch
(i) Zentrifugation bei 14.500 g für 45 Minuten, um alle Zellrückstände zu entfernen;
(ii) 0,2µ-Filtrieren des Überstandes gefolgt von 100 KD Diafiltration und Konzentration mit WFI (Water for Injection); und
(i) das Verfahren ausgeführt wird in einem kontinuierlich gefütterten Batch-Kultursystem.

3. Verfahren nach Anspruch 1, worin Schritt (e) umfasst:
(a) Entfernung von Protein- und Endotoxin-Verunreinigungen durch Verwendung eines anionischen Detergens mit einer Konzentration von 1 bis 1,2 % in Kombination mit einem Chelatbildner bei einer Konzentration von 1,5 bis 3 mM und Alkohol bei einer Konzentration von 40 % bis 50 %;
(b) Zugabe von Natriumacetat mit einer Konzentration von 4 bis 6 % zur Entfernung von Nukleinsäuren;
(c) Zugabe eines kationischen Detergens mit 3 bis 4 % zur Bindung von Polysaccharid und Verunreinigungen;
(d) Fällen von Polysaccharid aus dem kationischen Detergens-Polysaccharid-Komplex unter Verwendung von Natriumchlorid bei 0,05 M in Gegenwart von 96 % Alkohol;
(e) Entfernung von Protein- und Nukleinsäure-Verunreinigungen durch Waschen von Rückstand mit 45 % Alkohol in Gegenwart von Natriumchlorid bei einer Konzentration von 0,4 M;
(f) selektive Präzipitation von Polysaccharid unter Verwendung von 96 %igem Alkohol;
(g) Lösen von Polysaccharid in WFI und Unterwerfen einer Tangentialfluss-Filtration; und
worin dieses Reinigungsverfahren keinerlei Chromatographie verwendet.

4. Verfahren gemäß Anspruch 3, worin die Reinigung die folgenden Schritte umfasst:
(a) Entfernung von Protein- und Endotoxin-Verunreinigungen durch Verwendung von Deoxycholat bei einer Konzentration von 1 % in Kombination mit Ethylendiamintetraessigsäure bei einer Konzentration von 2 mM und Ethanol bei einer Konzentration von 40 %;
(b) Zugabe von 4 bis 6 % Natriumacetat zur Entfernung von Nukleinsäuren;
(c) Zugabe von Cetyltrimethylammoniumbromid bei einer Konzentration von 3 bis 4 % zur Bindung von Polysaccharid und Verunreinigungen;
(d) Fällen von Polysaccharid aus dem Cetyltrimethylammoniumbromid-Polysaccharid-Komplex unter Verwendung von Natriumchlorid bei einer Konzentration von 0,05 M in Gegenwart von 96 % Ethanol;
(e) Entfernung von Protein- und Nukleinsäure-Verunreinigungen durch Waschen des Rückstandes mit Ethanol bei einer Konzentration von 45 % in Gegenwart von Natriumchlorid bei einer Konzentration von 0,4 M;
(f) selektive Präzipitation von Polysaccharid unter Verwendung von 96 %igem Ethanol;
(g) Auflösen von Polysaccharid in WFI und Unterwerfen einer Tantentialfluss-Filtration; und
worin dieses Reinigungsverfahren keine Chromatographie einsetzt.

5. Verfahren gemäß Anspruch 1, weiter umfassend Schritt (h), worin gereinigtes N. meningitidis-X-Polysaccharid in eine durchschnittliche Größe von zwischen 100 und 150 kDa unter Verwendung von Hochdruckzelldisruptionssystemen gebracht wird.

6. Verfahren gemäß Anspruch 5,
weiter umfassend Schritt (i), worin dieses größenreduzierte Polysaccharid an ein Trägerprotein konkugiert wird, unter Bildung eines Protein-Saccharid-Konjugats.

7. Verfahren gemäß Anspruch 6,
worin dieses N. meningitidis-X-Saccharid an ein Trägerprotein konjugiert ist, welches ausgewählt ist aus der Gruppe bestehend aus TT, DT, CRM197, Fragment C von TT, Protein D, OMPC und Pneumolysin.

8. Verfahren gemäß Anspruch 6,
worin dieses N. menigitidis-X-Saccharid an ein Trägerprotein über einen hetero- oder homobifunktionellen Linker mit Cyanylierungs-Konjugations-Chemie gebunden wird.

9. Verfahren gemäß Anspruch 8, worin das Cyanylierungs-Reagenz ausgewählt ist aus einer Gruppe von 1-Cyano-4-pyrrolidinopyridinium-tetrafluorborat (CPPT), 1-Cyanoimidazol (1-Cl), 1-Cyanobenzotriazol (1-CBT), oder 2-Cyanopyridazin-3(2H)on (2-CPO).

## Revendications

1. Procédé de préparation d'un polysaccharide capsulaire d'un sérogroupe de Neisseria meningitidis (Neisseria meningitidis X) comprenant les étapes consistant en :
(a) la préparation d'un milieu nutritif aqueux de fermentateur comprenant des acides casamino,
(b) l'inoculation dans le milieu nutritif de fermentateur avec une bactérie Neisseria meningitidis X ayant une densité optique (DO) à 590 nm de 0,8 à 1,2 ;
(c) l'alimentation exponentielle continue avec une solution d'alimentation à une vitesse d'addition d'alimentation variant entre 10 ml/h/1,5 1 et 60 ml/h/1,5 1 en commençant à une DO entre 3 et 4 ;
(d) l'incubation du milieu nutritif de fermentateur inoculé dans des conditions de pH, de température et de pourcentage d'oxygène dissous contrôlées ;
(e) la récolte du saccharide capsulaire produit à l'étape (d) lorsque la densité optique (DO) à 590 nm est inférieure à 60 % de DO la plus élevée de la culture ;
(f) la purification du saccharide capsulaire obtenu à l'étape (e) ;
(g) éventuellement, la concentration du polysaccharide capsulaire purifié obtenu à l'étape (f) ;
et dans lequel ledit polysaccharide purifié a un rendement de 300 à 550 mg/l, un poids moléculaire moyen de 400 à 550 KDa, une récupération de l'étape de purification de 60 % à 65 % et contient moins de 0,5 % de protéines/peptides, moins de 0,5 % d'acides nucléiques et moins de 5 UE/µg d'endotoxines.

2. Procédé pour la production de polysaccharide capsulaire de Neisseria meningitidis X selon la revendication 1, dans lequel :
(a) ledit polysaccharide capsulaire produisant la bactérie est choisi parmi les souches de Neisseria meningitidis X M8210, M9601, M9591, M9592, M9554, M2526, 247X et 5967 (ST 750) ;
(b) ledit milieu nutritif de fermentateur comprend :
(i) des acides casamino en une quantité de 5 g/l à 20 g/l, 0,14 g/l à 0,19 g/l de chlorure d'ammonium, 10 g/l à 11 g/l de dextrose, 5,8 g/l à 6 g/l de chlorure de sodium, 0,9 g/l à 1 g/l de sulfate de potassium, 3,8 g/l à 4 g/l de phosphate de potassium dibasique, 4,8 g/l à 5 g/l d'acide glutamique, 0,2 g/l à 0,3 g/l d'arginine, 0,4 g/l à 0,5 g/l de L-sérine, 0,24 g/l à 0,25 g/l de cystéine, 0,18 g/l à 0,19 g/l de chlorure de magnésium, 0,02 g/l de chlorure de calcium et 0,002 g/l de sulfate ferreux de sorte que la concentration des composants du milieu puisse varier de ± 10 % ou
(ii) des acides casamino en une quantité de 5 g/l à 10 g/l, 0,14 g/l à 0,17 g/l de chlorure d'ammonium, 10 g/l à 11 g/l de dextrose, 5,8 g/l à 6 g/l de chlorure de sodium, 0,9 g/l à 1 g/l de sulfate de potassium, 3,8 g/l à 4 g/l de phosphate de potassium dibasique, 4,8 g/l à 5 g/l d'acide glutamique, 0,2 g/l à 0,3 g/l de L-arginine, 0,4 g/l à 0,5 g/l de L-sérine, 0,24 g/l à 0,25 g/l de L-cystéine, 0,18 g/l à 0,19 g/l de chlorure de magnésium, 0,02 g/l de chlorure de calcium et 0,002 g/l de sulfate ferreux de sorte que la concentration des composants du milieu puisse varier de ± 10 %,
(c) ladite inoculation du milieu nutritif du fermentateur est un inoculum d'ensemencement ayant une densité optique de 0,8 à 1 ;
(d) ladite alimentation exponentielle continue commence avec un apport d'alimentation initial à une vitesse de 10 ml/h/1,5 l lorsque la DO à 590 nm se situe entre 3 et 4, qui augmente progressivement à 30 ml/h/1,5 l jusqu'à ce que la DO de la culture soit la plus élevée puis est maintenue à 60 ml/h/1,5 l jusqu'à ce que la DO de la culture atteigne 50 % de DO la plus élevée lorsque la culture peut être récoltée ;
(e) ladite solution d'alimentation comprend de 72 à 76 g/l de dextrose, 38 g/l à 42 g/l de glutamate de sodium, 2,8 g/l à 3,2 g/l de L-arginine, 2,8 g/l à 3,2 g/l de L-sérine, 1,9 g/l à 2,1 g/l de L-cystéine, 1,9 g/l à 2 g/l de chlorure de magnésium, 0,13 g/l à 0,15 g/l de chlorure de calcium et 0,02 g/l de sulfate ferreux de sorte que cette concentration des composants de la solution d'alimentation puisse varier de ± 10 %,
(f) ladite incubation est réalisée
(i) à une température de 36 °C à 37 °C, à un pH de 7 à 7,2, à 350 à 500 t/m, un pourcentage d'oxygène dissous de 15 % à 25 %, un flux de gaz de 1 à 1,5, de l'air à 0 à 100 % et de l'oxygène à 0 à 100 % pendant 7 à 10 heures ou
(ii) à une température de 36 °C, à un pH de 7,2, à environ 400 t/m, un pourcentage d'oxygène dissous de 25 %, un flux de gaz de 1 à 1,5,
de l'air à 0 à 100 % et de l'oxygène à 0 à 100 % pendant 8 à 9 heures ;
(g) l'inactivation est réalisée en utilisant 1 % de formaldéhyde pendant 1 à 2 h à 37 °C suivie d'une incubation à 10 °C pendant 30 minutes ;
(h) ladite récolte est réalisée par
(i) centrifugation à 14500 g pendant 45 minutes pour éliminer tous les résidus cellulaires ;
(ii) soumission du surnageant à une filtration à 0,2 µ suivie d'une diafiltration à 100 KD et une concentration avec de l'EPI (eau pour injection) ; et
(i) ledit procédé est réalisé dans un système de culture fed-batch en continu.

3. Procédé selon la revendication 1, dans lequel l'étape (e) comprend :
(a) le retrait d'impuretés sous forme de protéines et d'endotoxines en utilisant un détergent anionique en une concentration de 1 à 1,2 % en combinaison avec un agent chélateur à une concentration de 1,5 à 3 mM et de l'alcool à une concentration de 40 % à 50 % ;
(b) l'addition d'acétate de sodium en une concentration de 4 à 6 % pour le retrait des acides nucléiques ;
(c) l'addition d'un détergent cationique à 3 à 4 % pour lier le polysaccharide et les impuretés ;
(d) la précipitation du polysaccharide à partir du complexe détergent cationique-polysaccharide en utilisant du chlorure de sodium à 0,05 M en présence d'alcool à 96 % ;
(e) le retrait des impuretés sous forme de protéine et d'acide nucléique par lavage du culot avec de l'alcool à 45 % en présence de chlorure de sodium en une concentration de 0,4 M ;
(f) la précipitation sélective du polysaccharide en utilisant de l'alcool à 96 % ;
(g) la dissolution du polysaccharide dans de l'EPI et sa soumission à une filtration à flux tangentiel ; et
dans lequel ledit processus de purification n'utilise pas de chromatographie

4. Procédé selon la revendication 3, dans lequel la purification comprend les étapes suivantes :
(a) le retrait des impuretés sous forme de protéines et d'endotoxines en utilisant du désoxycholate en une concentration de 1 % en combinaison avec de l'acide éthylène-diamine-tétra-acétique en une concentration de 2 mM et de l'éthanol en une concentration de 40 %,
(b) l'addition de 4 à 6 % d'acétate de sodium pour le retrait des acides nucléiques ;
(c) l'addition de bromure de cétyltriméthylammonium en une concentration de 3 à 4 % pour lier le polysaccharide et les impuretés ;
(d) la précipitation du polysaccharide à partir du complexe bromure de cétyltriméthylammonium - polysaccharide en utilisant du chlorure de sodium en une concentration de 0,05 M en présence d'éthanol à 96 % ;
(e) le retrait des impuretés sous forme de protéines et d'acide nucléique en lavant le culot avec de l'éthanol en une concentration de 45 % en présence de chlorure de sodium en une concentration de 0,4 M ;
(f) la précipitation sélective du polysaccharide en utilisant de l'alcool à 96 % ;
(g) la dissolution du polysaccharide dans de l'EPI et sa soumission à une filtration à flux tangentiel ; et
dans lequel ledit processus de purification n'utilise pas de chromatographie

5. Procédé selon la revendication 1, comprenant en outre l'étape (h) dans laquelle le polysaccharide de N. meningitidis purifié est dimensionné à une taille moyenne entre 100 et 150 kDa en utilisant un système de rupture cellulaire à haute pression.

6. Procédé selon la revendication 5, comprenant en outre l'étape (i) dans laquelle ledit polysaccharide à taille réduite est conjugué à une protéine de support pour donner un conjugué protéine-saccharide.

7. Procédé selon la revendication 6, dans lequel ledit saccharide de N. meningitidis X est conjugué à une protéine de support choisie dans le groupe consistant en TT, DT, CRM197, le fragment C de TT, la protéine D, OMPC et pneumolysine.

8. Procédé selon la revendication 6, dans lequel ledit saccharide de N. meningitidis X est conjugué à la protéine de support via un segment de liaison hétéro- ou homo-bifonctionnel avec une chimie de conjugaison par cyanylation.

9. Procédé selon la revendication 8, dans lequel ledit réactif de cyanylation est choisi dans le groupe comprenant le tétrafluoroborate de I-cyano-4-pyrrolidinopyridinium (CPPT), le 1-cyano-imidazole (1-Cl), le 1-cyanobenzotriazole (1-CBT) ou la 2-cyanopyridazine-3(2H)-one (2-CPO).
